(19) Europäisches Patentamt
European Patent Office
Office européen des brevets

(11) **EP 1 391 703 B1**

(12) **EUROPÄISCHE PATENTSCHRIFT**

(45) Veröffentlichungstag und Bekanntmachung des Hinweises auf die Patenterteilung:
**24.01.2007 Patentblatt 2007/04**

(51) Int Cl.:
*G01F 15/04* *(2006.01)*    *G01F 15/00* *(2006.01)*
*G01F 1/68* *(2006.01)*    *G01F 25/00* *(2006.01)*

(21) Anmeldenummer: **02405715.0**

(22) Anmeldetag: **22.08.2002**

(54) **Thermisches Gasdurchfluss-Messgerät mit Gasqualitätsindikator**

Thermal gas flow measuring device with gas quality indicator

Dispositif thermique de mesure du débit de gaz avec indicateur de qualité du gaz

(84) Benannte Vertragsstaaten:
**AT BE BG CH CY CZ DE DK EE ES FI FR GB GR IE IT LI LU MC NL PT SE SK TR**

(43) Veröffentlichungstag der Anmeldung:
**25.02.2004 Patentblatt 2004/09**

(73) Patentinhaber: **EMS-PATENT AG**
**7013 Domat/Ems (CH)**

(72) Erfinder:
• **Matter, Daniel**
**5200 Brugg (CH)**
• **Luchsinger, Rolf**
**5416 Kirchdorf (CH)**
• **Kramer, Beat**
**5210 Windisch (CH)**

• **Sabbattini, Bruno**
**5430 Wettingen (CH)**

(74) Vertreter: **Reitzle, Helmut**
**Pfenning, Meinig & Partner GbR**
**Patent- und Rechtsanwälte**
**Theresienhöhe 13**
**80339 München (DE)**

(56) Entgegenhaltungen:
**EP-A- 0 468 793**    **EP-A- 1 164 361**
**WO-A-01/18500**    **FR-A- 2 776 776**
**US-A- 4 345 463**

• **PATENT ABSTRACTS OF JAPAN vol. 1999, no. 09, 30. Juli 1999 (1999-07-30) & JP 11 118569 A (YAZAKI CORP), 30. April 1999 (1999-04-30)**

**Beschreibung**

**TECHNISCHES GEBIET**

Die vorliegende Erfindung bezieht sich auf das Gebiet der Messung von Gasströmungen mit thermischen Sensoren. Sie geht aus von einem Verfahren und einem Sensor zur Massenflussmessung gemäss Oberbegriff der unabhängigen Ansprüche.

**STAND DER TECHNIK**

In der WO 01/96819 A1 wird ein Gaszähler offenbart, der als Energiemessgerät geeicht ist. Die Eichung beruht darauf, dass Sensorsignalwerte in Abhängigkeit der Durchflussrate eines Eichgases oder Kalibriergases bestimmt und in Form einer Sensoreichkurve oder Sensorkalibrierkurve im Gaszähler gespeichert werden. Die Sensoreichkurve beziehungsweise die Sensorsignalwerte werden mit einem Signal-Umrechnungsfaktor und einem Brennwertfaktor für das Basis-Gasgemisch multipliziert, so dass das erhaltene Produkt einen Gasverbrauch in einer Leistungseinheit und nach Integration in einer Energieeinheit angibt. Mit einem weiteren Korrekturfaktor kann wenigstens näherungsweise der tatsächliche Heizwert eines bezogenen Gasgemisches in der Energieeichung berücksichtigt werden. Als tatsächlicher Heizwert kann ein gemessener, über eine bestimmte Zeitspanne gemittelter Heizwert verwendet werden. Nachteilig ist, dass eine externe Einheit zur Bestimmung des Heizwerts erforderlich ist.

In der EP 0 373 965 werden ein Verfahren und eine Vorrichtung zur Bestimmung eines Gas- oder Energieverbrauchs aus einem korrigierten Massenflusssignal offenbart. Bei der Signalkorrektur werden die Wärmeleitfähigkeit, spezifische Wärmekapazität und Dichte des Gases berücksichtigt. Das korrigierte Massenflusssignal und damit der Gas- oder Energieverbrauch sind unabhängig von der Gasart und insbesondere identisch für Luft, Argon, Helium, Kohlendioxid, Methan und Propan. Nachteilig ist, dass ein solcherart normiertes Massenflusssignal insensitiv für den Heizwert eines Gases oder Gasgemisches ist, da brennbare Gase mit unterschiedlichem Heizwert (z. B. Methan oder Propan) gleiche Massenflusssignale und sogar gleiche Signale wie unbrennbare Gase (z. B. Helium, Argon, Kohlendioxid oder Luft) ergeben.

In dem U. S. Pat. No. 5'311'447 werden ein Verfahren und eine Vorrichtung zur verbrennungslosen Bestimmung des spezifischen Heizwerts von Erdgas offenbart. Hierzu werden mit empirischen Formeln spezifischer Heizwert, Dichte oder Anteil inerter Gase aus gemessenen Werten von Viskosität, Wärmeleitfähigkeit, Wärmekapazität, optischer Absorption usw. bestimmt. Nachteilig ist der grosse Mess- und Rechenaufwand bei der quantitativen Messung mehrerer unabhängiger gasartabhängiger Grössen und bei deren Zusammenführung mit einer Volumenflussmessung in einem Gaszähler zur Bestimmung einer konsumierten Energiemenge.

In der WO 01/18500 wird eine verbesserte Massenflussmessung mit zwei thermischen CMOS-Anemometern offenbart. Am ruhenden Gas werden bei konstanter Heizleistung eine Wärmeleitfähigkeit und bei gepulster Heizleistung eine Wärmekapazität gemessen, das Gas identifiziert und aus dessen spezifischen Heizwert zusammen mit der Massenflussmessung der totale Brennwert des Gases bestimmt. Nachteilig ist wiederum der relativ grosse Aufwand bei der Bestimmung der konsumierten Energiemenge aus separaten Werten von Massenfluss und spezifischem Heizwert. Zudem muss der spezifische Heizwert für eine hinreichend genaue Bestimmung des Energiebezugs kontinuierlich und mit grosser Genauigkeit gemessen werden.

**DARSTELLUNG DER ERFINDUNG**

Aufgabe der vorliegenden Erfindung ist es, ein Verfahren und eine Vorrichtung zur Bestimmung einer Durchflussrate anzugeben, wobei eine verbesserte Eichbarkeit erreicht wird. Diese Aufgabe wird erfindungsgemäss durch die Merkmale der unabhängigen Ansprüche gelöst.

In einem ersten Aspekt besteht die Erfindung in einem Verfahren zum Messen eines Gasverbrauchs mittels eines Gaszählers, insbesondere zum Messen eines verrechenbaren Gasenergiebezugs im privaten, öffentlichen oder industriellen Bereich, wobei vom Gaszähler mit Hilfe eines thermischen Durchflusssensors zu einer Durchflussrate im wesentlichen proportionale Sensorsignalwerte bestimmt werden und die Sensorsignalwerte aufgrund einer Kalibration des Gaszählers als Energiemessgerät als Energiewerte ausgegeben werden, wobei vom Gaszähler eine Gasart insoweit bestimmt wird, dass ein nicht brennbares Gasgemisch von einem brennbaren Gasgemisch unterschieden wird und der Gaszähler bei Vorhandensein eines nicht brennbaren Gasgemisches mit einer Kalibration in Massen- oder Normvolumeneinheiten und bei Vorhandensein eines brennbaren Gasgemisches mit einer Kalibration in Energieeinheiten betrieben wird. Der Betrieb als Energiemessgerät umfasst auch Kalibration und Betrieb als Leistungsmessgerät mit Ausgabe von Leistungswerten. Das erfindungsgemässe Verfahren und Gasmeter bringt diverse Vorteile. Die Zuverlässigkeit der Energiemessung wird deutlich erhöht, da mit geringem Aufwand beim durchströmenden Gas streng unterschieden wird zwischen hochwertigem Nutzgas und nicht brennbarem Gas. Insbesondere wird automatisch zwischen einem nicht brennbaren Eichgas, typischerweise Stickstoff oder Luft, und einem Basis-Gasgemisch oder zu messenden Gas unterschieden und eine automatische Umschaltung von einer Massen- oder Volumenskala auf eine Energieskala durchgeführt. Die gleiche Unterscheidung wird auch bei einer Ausserbetriebnahme, Inbetriebnahme, bei Manipulation am Gaszähler oder aus anderem Grund wirksam, so dass Verfälschungen der Energiemessung durch Kontakt mit Luft o. ä. ausgeschlossen sind. Der Betrieb mit einer Kalibration in Massen-, Volumen- oder Energieeinheiten beinhaltet insbesondere eine Signalausgabe und/oder Signalanzeige in diesen Einheiten.

In einem ersten Ausführungsbeispiel wird mit Hilfe eines thermischen Gasqualitätssensors mindestens ein gasartabhängiger Parameter des Gasgemisches, insbesondere ein Wärmekoeffizient wie z. B. eine Wärmeleitfähigkeit $\lambda$ und/oder Wärmekapazität c oder eine Viskosität $\eta$, bestimmt und durch Vergleich mit bekannten Werten des Parameters für bekannte Gase oder Gasgemische das Gasgemisch als brennbar oder nicht brennbar identifiziert. Es genügt also eine ungefähre Kenntnis der Art, oder Zusammensetzung des Gases, damit eine digitale Entscheidung brennbar/unbrennbar getroffen und die entsprechende Kalibrierung aktiviert werden kann.

Das Ausführungsbeispiel gemäss Anspruch 3 hat den Vorteil einer besonders einfachen Sensorkonfiguration und Signalauswertung. Eine Summenbildung der Temperatursignale bewirkt, dass das Signal zur Bestimmung eines gasartspezifischen Parameters oder Wärmekoeffizienten unabhängig von der Flussrichtung und von möglichen Asymmetrien der Anordnung der Temperatursensoren ist. Es wird auch ein grösseres Signal erzielt als bei Verwendung des stromaufwärts gelegenen Temperatursensors alleine.

Die Ausführungsbeispiele gemäss Anspruch 4 und 5 haben den Vorteil, dass eine einfache Rechenvorschrift genügt, um das anwesende Gas oder Gasgemisch mit hoher Zuverlässigkeit als brennbar und damit für einen verrechenbaren Energiebezug geeignet oder als nicht brennbar und damit als nicht verrechenbaren Massenfluss zu kategorisieren.

Die Ausführungsbeispiele gemäss Anspruch 6 haben den Vorteil, dass der Strombedarf des Gaszählers ohne Verlust an Messgenauigkeit wirkungsvoll gesenkt werden kann.

Das Ausführungsbeispiel gemäss Anspruch 7 hat den Vorteil, dass der gesamte Gasenergieverbrauch oder Energiebezug auch dann korrekt bestimmt werden kann, wenn Umschaltungen zwischen der Kalibration in Energieeinheiten und anderen Durchflusseinheiten wie Masse oder Volumen durchgeführt wurden.

Das Ausführungsbeispiel gemäss Anspruch 8 hat den Vorteil, dass die Durchflussmessung in Massen- oder Normvolumeneinheiten wahlweise ununterbrochen fortgeführt wird, z. B. um einen Gesamtvolumenfluss zu bestimmen, oder nur bei Fluss unbrennbarer Gase aufintegriert wird, z. B. um bei geschlossenem Gaskreislauf eine komplementäre Kontrollgrösse für den Bezug brennbarer Gase zu generieren, oder nach jeder Umschaltung der Kalibration neu initialisiert wird, um Unterbrüche beim Energiebezug zu dokumentieren.

Ausführungsbeispiele gemäss Anspruch 9 haben insbesondere den Vorteil, dass Manipulationsversuche am Gaszähler einfach erkennbar sind.

Das Ausführungsbeispiel gemäss Anspruch 10 hat den Vorteil, dass eine automatische Heizwertnachführung auch ohne irgendeine externe oder interne Bestimmung des aktuellen spezifischen Heizwerts des Gases oder Gasgemisches durchgeführt wird.

In einem zweiten Aspekt besteht die Erfindung in einem Gaszähler mit einem thermischen Massenflusssensor zum Ermitteln eines Gasenergiebezugs gemäss dem zuvor beschriebenen Verfahren. Der Gaszähler umfasst einen thermischen Durchflusssensor, ist als Energiemessgerät in Energieeinheiten und zusätzlich als Massenflussmeter in Massen- oder Normvolumeneinheiten kalibriert, weist einen Gasqualitätssensor auf, der ein Diskriminationssignal, insbesondere einen gasartabhängigen Parameter oder Wärmekoeffizienten, zur Unterscheidung eines brennbaren Gasgemisches von einem nicht brennbaren Gasgemisch erzeugt, und ist aufgrund des Diskriminationssignals zwischen einem Betrieb als Energiemessgerät oder als Massenflussmeter umschaltbar. Der Gaszähler ist also für Eichzwecke, bei Lagerung oder bei Ausserbetriebnahme als Massenflussmeter oder, mit zusätzlicher Dichtemessung, als Volumenflussmeter kalibriert und für Mess- oder Verrechnungszwecke als Energiemeter. Im Betrieb findet keine Verrechnung statt, wenn Luft detektiert wird. Stattdessen kann eine Durchflussmessung in Masse oder Volumen durchgeführt werden.

Die Ausführungsbeispiele gemäss Ansprüchen 12-15 ermöglichen einen besonders einfachen Aufbau und Betrieb des Gaszählers. Insbesondere sind Manipulationsversuche am Gaszähler im Betrieb erkennbar, wenn eine wiederkehrende Kontaktnahme mit Luft detektiert wird.

Weitere Ausführungen, Vorteile und Anwendungen der Erfindung ergeben sich aus abhängigen Ansprüchen sowie aus der nun folgenden Beschreibung und den Figuren.

## KURZE BESCHREIBUNG DER ZEICHNUNG

Es zeigen:

Fig. 1    im Querschnitt ein durchströmtes Rohr mit einem thermischen Durchflusssensor, der Bestandteil eines Gaszählers mit erfindungsgemäss dualer Kalibrierung als Energie- und Mengenzähler ist;

Fig. 2    Temperatursummensignale zur Bestimmung gasspezifischer Wärmeübergangskoeffizienten;

Fig. 3    eine Kalibrationskurve für den Übergang zwischen Eichgas und Basis-Gasgemisch (Nutzgas); und

Fig. 4    eine Tabelle mit Gasparametern für Erdgas.

In den Figuren sind gleiche Teile mit gleichen Bezugszeichen versehen.

## WEGE ZUR AUSFÜHRUNG DER ERFINDUNG

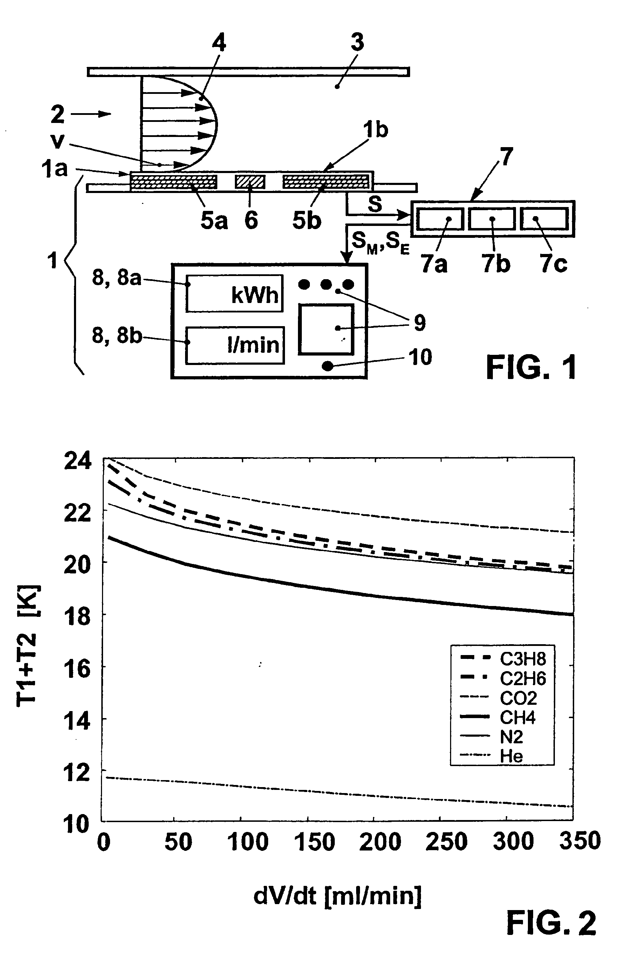

Fig. 1 zeigt einen Gaszähler 1 umfassend einen thermischen Durchfluss- oder Massenflusssensor 1a, 1b, 7, der ein in einem Strömungskanal oder Rohr 2 angeordnetes Sensorelement 1a, eine Membran 1b und eine Mess- und Auswerteeinheit 7 umfasst. Im Rohr 2 strömt ein Gas 3 mit einem Strömungs- oder Geschwindigkeitsprofil 4. Das Sensorelement 1a ist einer zu messenden Strömungsgeschwindigkeit v ausgesetzt. Der Durchflusssensor 1 umfasst ein Heizelement 6, stromaufwärts einen ersten Temperatursensor 5a und stromabwärts einen zweiten Temperatursensor 5b. Aus Temperatursignalen $T_1$, $T_2$ der Temperatursensoren 5a, 5b kann bekanntermassen ein Massenfluss- oder Normvolumenflusssignal $S_M$ bestimmt werden. Die prinzipielle Funktionsweise beruht darauf, dass eine vom Heizelement 6 erzeugte Temperaturverteilung durch die Strömung 4 asymmetrisch wird und ein Temperaturunterschied $T_1$-$T_2$ an den Temperatursensoren 5a, 5b als Mass für die Strömungsgeschwindigkeit v oder den Massenfluss dm/dT verwendet wird. In guter Näherung gilt Massenflusssignal $S_M$ proportional zu Temperaturdifferenz $T_1$-$T_2$. Im vorliegenden Fall werden zudem durch die Messmittel 7 aus den Massenflusssignalen $S_M$ oder allgemein Sensorsignalen S des Durchflusssensors 1a aufgrund einer Kalibration des Gaszählers 1 als Energiemessgerät Energiewertsignale $S_E$ bestimmt und ausgegeben. Die Kalibration als Energiemessgerät ist in der WO 01/96819 A1 offenbart, deren Inhalt hiermit in vollem Umfang durch Bezugnahme in die vorliegende Offenbarung aufgenommen wird. Ebenso seien die darin zitierten drei Artikel zum CMOS-Anemometer von J. Robadey sowie F. Mayer et al. durch Bezugnahme hier aufgenommen. Das dort beschriebene CMOS-Anemometer ist besonders geeignet als Sensorelement 1a des Durchflusssensors.

Erfindungsgemäss wird nun vom Gaszähler 1 eine Gasart insoweit bestimmt, dass ein nicht brennbares Gasgemisch 3 von einem brennbaren Gasgemisch 3 unterschieden wird und der Gaszähler 1 bei Vorhandensein eines nicht brennbaren Gasgemisches 3 mit einer Kalibration in Massen- oder Normvolumeneinheiten, z. B. l/min, und bei Vorhandensein eines brennbaren Gasgemisches 3 mit einer Kalibration in Energie- oder Leistungseinheiten, z. B. kWh, betrieben wird.

Für die Funktionsfähigkeit des Gaszählers 1 als Energie- und Massenflussmeter kann statt des Durchflusssensors 1a mit zwei Temperatursensoren 5a, 5b und insbesondere statt des CMOS-Anemometers 1a auch allgemein ein thermischer Durchflusssensor verwendet werden, bei welchem das Gas 3 über ein Sensorelement geführt wird, welches ein Heizmittel zur Temperaturänderung und ein Sensormittel zur Bestimmung seiner Temperatur aufweist, wobei die flussabhängige Temperaturänderung wiederum ein Mass für den Massenfluss ist. Alternativ kann der thermische Durchflusssensor 1a auch mit nur einem stromabwärts angeordneten Temperatursensor 5a betrieben werden. Generell kann der Massenfluss dm/dt in Massen- oder Normvolumeneinheiten, z. B. in kg/min, angegeben werden oder mit Hilfe der Dichte $\rho$ aus einem Volumenfluss dV/dT bestimmt werden gemäss dm/dt=$\rho$*dV/dT. Im Gaszähler 1 bedeutet eine Signalausgabe eine Signalanzeige und/oder Signalübertragung an eine Ablese- oder zentrale Auswerteeinheit (nicht dargestellt).

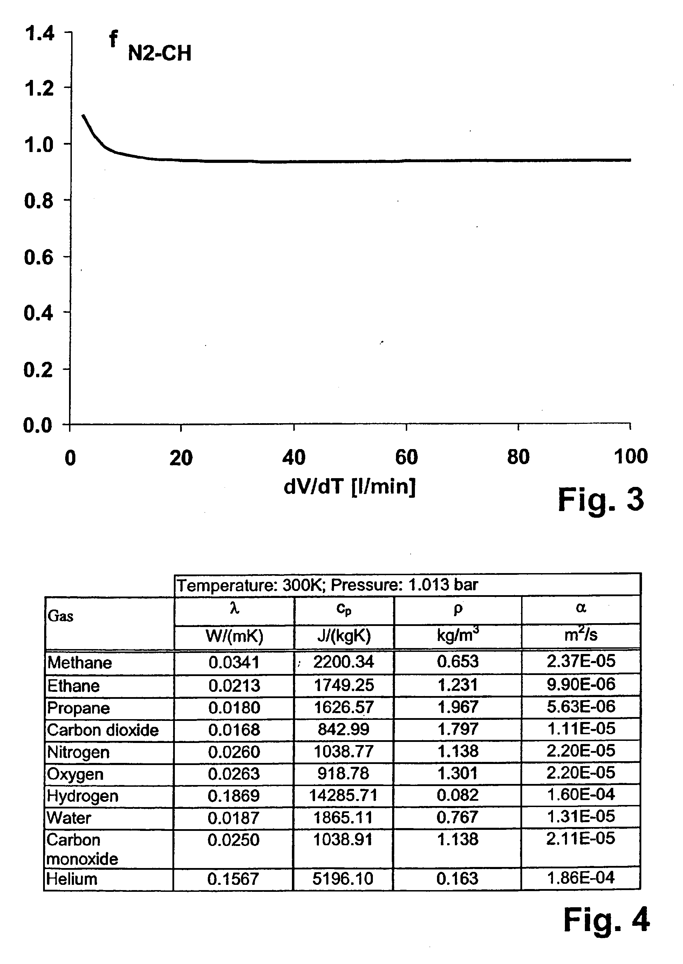

Gemäss der WO 01/96819 A1 wird mit einem Eichgas 3, typischerweise Stickstoff $N_2$ oder Luft, ein Sensorsignal S gemessen, das im wesentlichen proportional zur Normvolumen-Durchflussrate $d(V_{N2,n})$/dt des Eichgases 3 ist. Durch Inversion von $S(d(V_{N2,n})$/dt wird eine Sensoreichkurve F(S) (Durchflussrate in Abhängigkeit des Sensorsignals S), vormals mit $F_n(S(d(V_{N2,n})$/dt bezeichnet, bestimmt und in der Auswerteeinheit 7 des Gaszählers 1 abgespeichert. Im Betrieb wird dann das Sensorsignal S mit Hilfe der Sensoreichkurve F(S) auf ein (unkorrigiertes) Massenflusssignal $S_m$ kalibriert, welches proportional zu F(S) ist oder einfach $S_m$=F(S) ist. Die Kalibration der Durchflussrate kann also durch eine Sensoreichkurve F(S) für das Eichgas unter Normbedingungen ausgedrückt werden. Das Massenflussratensignal $S_m$ hängt noch von der Gassorte ab. Daher werden Abweichungen des Massenflussratensignals $S_m$ von einem exakten Idealwert für ein Basis-Gasgemisch, typischerweise Erdgas oder allgemein ein Kohlenwasserstoffgemisch CH, durch einen Signal-Umrechnungsfaktor oder Sensorsignal-Korrekturfaktor $f_{N2-CH}$ korrigiert (Fig. 3). Somit gilt $S_M$=$S_m$*$f_{N2-CH}$ mit $S_M$=korrigiertes Massenflussratensignal. Schliesslich wird ein Energiewertsignal $S_E$ durch Multiplikation mit einem Heizwert $H_{CH}$ (kalorimetrischer Wert pro Einheit der Durchflussgrösse, d. h. pro Standardvolumen oder pro Masse) des Basis-Gasgemisches und Integration bestimmt: $S_E = \int S_M \bullet H_{CH} \bullet dt = f_{N2-CH} \bullet H_{CH} \bullet \int F(S) \bullet dt$.

Ausgehend von dieser Energiekalibration für das Basis-Gasgemisch CH ist es nun jedoch nicht mehr notwendig, am Gasgemisch eine Messung des aktuellen Heizwerts des Gasgemisches durchzuführen. Gemäss der WO 01/96819 A1 erfolgt nämlich im thermischen Durchflusssensor 1a, insbesondere im CMOS-Anemometer-Durchflusssensor 1a, eine inhärente automatische Heizwertnachführung bei Abweichungen des aktuellen Gasgemisches 3 vom Basis-Gasgemisch CH. Es genügt also, eine ungefähre Kenntnis über Art und/oder Zusammensetzung des Gases 3 zu erlangen und eine digitale Entscheidung herbeizuführen, ob ein brennbares oder verrechenbares Gas 3 bezogen wird oder aber nur ein Durchfluss eines nicht brennbaren oder zumindest nicht verrechenbaren Gasbezugs gemessen werden soll, wobei im ersten Fall ohne Heizwertmessung eine relativ zuverlässige, auf den aktuellen Heizwert bezogene Energiemessung erfolgt.

Gemäss der WO 01/96819 A1 oder der unveröffentlichten EP-Anmeldung Nr. 01 810 546.0, hiermit in vollem Umfang durch Bezugnahme aufgenommen, können für die genannten Grössen S, F(S), $f_{N2-CH}$ und $H_{CH}$ und daraus ableitbare Grössen auch geeignete Zeitmittelwerte verwendet werden.

Bevorzugt wird mit Hilfe eines thermischen Gasqualitätssensors 1a mindestens ein gasartabhängiger Parameter $\lambda$, c, $\alpha$ (Diffusivität), $\eta$ (Viskosität) des Gasgemisches 3, insbesondere ein Wärmekoeffizient $\lambda$, c, $\alpha$ wie z. B. eine Wärmeleitfähigkeit $\lambda$ und/oder eine Wärmekapazität c, bestimmt und durch Vergleich mit bekannten Werten des Parameters $\lambda$, c, $\alpha$, $\eta$ für bekannte Gase oder Gasgemische das Gasgemisch 3 als brennbar oder nicht brennbar identifiziert.

Im folgenden wird eine detailliertere Analyse zur Messung der Wärmeleitfähigkeit mit dem thermischen Durchflusssensor 1a gegeben. Das zu messende Gas 3 kann als weitgehend inkompressibel angenommen werden, da relative Dichteänderungen $\Delta\rho/\rho \approx 1/2(v/c_0)^2$ mit v = Flussgeschwindigkeit und $c_0$ = Schallgeschwindigkeit für typische Werte $v \approx$ 3 m/s und $c_0 \approx$ 300 m/s im Bereich von $10^{-4}$ liegen und somit vernachlässigbar sind. Für inkompressible Gase 3, d. h. $v << c_0$, und unter Vernachlässigung viskoser Dissipation kann der Wärmetransport inklusive Konvektion aus der stationären Wärmeleitungsgleichung durch Hinzufügen eines konvektiven Terms hergeleitet werden. Für einen Strömungskanal 2 in x-Richtung ohne Wärmequelle im Gas 3 lautet die Wärmeleitungsgleichung mit erzwungener Konvektion

$$\lambda \cdot \left(\frac{\partial^2 T}{\partial x^2} + \frac{\partial^2 T}{\partial y^2} + \frac{\partial^2 T}{\partial z^2}\right) = v_x \cdot c_p \cdot \rho \cdot \frac{\partial T}{\partial x} \qquad \text{(G1)}$$

mit T = T(x, y, z) das stationäre Temperaturfeld im Gas 3, $\lambda$ = Wärmeleitfähigkeit, $v_x$ = Flussgeschwindigkeit in x-Richtung, $c_p$ = Wärmekapazität und $\rho$ = Dichte des Gases 3. Für vernachlässigbare Konvektion $v_x \approx 0$ kann die Wärmeleitfähigkeit $\lambda$ bestimmt werden, indem die stationäre Diffusionsgleichung

$$\lambda \cdot \left(\frac{\partial^2 T}{\partial x^2} + \frac{\partial^2 T}{\partial y^2} + \frac{\partial^2 T}{\partial z^2}\right) = 0 \qquad \text{(G2)}$$

integriert wird und die korrekten Randbedingungen für die Integrationskonstanten (Wärmestrom j≠0, keine Wärmequelle im Gas 3) eingesetzt werden. Für nicht vernachlässigbare Konvektion $v_x > 0$ kann aus Gleichung (G1) bei bekanntem $v_x$ die inverse thermische Diffusivität $\alpha^{-1} = c_p\rho/\lambda$ bestimmt werden.

Gleichung (G1) wurde mit einer finiten Elemente Berechnung für den Durchflusssensor 1a gemäss Fig. 1 in CMOS-Ausführung für typische Gasbestandteile von Erdgas (Propan $C_3H_8$, Äthan $C_2H_6$, Kohlendioxid $CO_2$, Methan $CH_4$, Stickstoff $N_2$ und Helium He) unter Verwendung von deren bekannten Wärmekoeffizienten $\lambda$, $c_p$, $\alpha$ gelöst. In Fig. 2 ist die resultierende Temperatursumme $T_1 + T_2$ für diese Erdgaskomponenten als Funktion der Flussgeschwindigkeit $v_x$ aufgetragen. Die Temperatursumme $T_1 + T_2$ für kleine $v_x$ (im ungefähren Bereich 0...20 ml/min, insbesondere 0...5 ml/min) sind deutlich unterscheidbar, da die zugrundeliegenden Wärmeleitfähigkeiten $\lambda$ (s. Fig. 4) hinreichend unterschiedliche Werte haben. Es genügt also, am herkömmlichen thermischen Durchflusssensor 1a einfach ein Summensignal der Temperatursensoren 5a, 5b als Mass für eine Gasart und insbesondere als Diskriminationssignal zur Unterscheidung zwischen einem brennbaren und unbrennbaren oder nicht verrechenbaren Gas 3 zu verwenden. Auch aus dem Temperatursignal des ersten Temperatursensors 5a alleine - und sogar aus dem weniger variierenden Temperatursignal des zweiten Temperatursensors 5b alleine - kann ein gasartabhängiger Wärmekoeffizient $\lambda$, c, $\alpha$ bestimmt werden. Insbesondere kann aufgrund des Wärmetransports in Flussrichtung immer bestimmt werden, welcher Temperatursensor 5a, 5b der erste, d. h. stromaufwärts gelegene und welcher der zweite, d. h. stromabwärts gelegene ist. Auch für grössere Flussgeschwindigkeiten $v_x >> 0$ sind die Temperaturkurven $T_1 + T_2$ oder $T_1$ alleine (nicht dargestellt) gasartabhängig und unterscheidbar, da die zugrundeliegenden Diffusivitätswerte $\alpha$ und/oder Wärmekapazitäten $c_p$ oder allgemein c unterschiedlich sind. Gemäss der WO 01/18500 können auch, wie eingangs erwähnt, am ruhenden Gas bei konstanter Heizleistung die Wärmeleitfähigkeit $\lambda$ und separat davon bei gepulster Heizleistung die Wärmekapazität c oder c*$\rho$ gemessen werden. Hierfür wird mindestens zeitweise das Heizmittel mit einer konstanten Heizleistung oder in Form von Heizpulsen betrieben und eine strömungsunabhängige Wärmeleitfähigkeit $\lambda$ oder Wärmekapazität c ge-messen.

Fig. 4 zeigt eine Tabelle mit Wärmekoeffizienten $\lambda$, $c_p$, $\alpha$ und spezifischen Dichten $\rho$ typischer Erdgasbestand-

teile Methan, Äthan, Propan, Sauerstoff, Wasserstoff, Kohlenmonoxid (brennbar) und Kohlendioxid, Stickstoff, Wasser und Helium (unbrennbar). In einem bevorzugten Ausführungsbeispiel wird eine gemessene Wärmeleitfähigkeit $\lambda$ auf Übereinstimmung mit einem Wärmeleitfähigkeitswert entsprechend einem Absolutwert von 0,026 W/mK für Stickstoff, Sauerstoff oder Luft, insbesondere 0,0260 W/mK für Stickstoff, 0.0263 W/mK für Sauerstoff oder 0,0261 W/mK für Luft, oder 0,0168 W/mK für Kohlendioxid getestet, wobei eine vorgebbare Toleranz von $\pm 10\%$, bevorzugt $\pm 5\%$ und besonders bevorzugt $\pm 2\%$ berücksichtigt wird. Bei Übereinstimmung wird das Gasgemisch 3 als nicht brennbar kategorisiert und eine Signalausgabe 8 des Gaszählers 1 mit einer in Massen- oder Normvolumeneinheiten, z. B. l/min, kalibrierten Skala 8b betrieben. Bei Nichtübereinstimmung wird das Gasgemisch 3 als brennbar kategorisiert und eine Signalausgabe 8 des Gaszählers 1 mit einer in Energieeinheiten, z. B. kWh, kalibrierten Skala 8a betrieben.

Alternativ oder ergänzend wird eine gemessene Wärmekapazität c oder $c_p$ mit einem Schwellwert entsprechend einem Absolutwert von 1300 J/kgK verglichen, wobei eine vorgebbare Toleranz von $\pm 10\%$, bevorzugt $\pm 5\%$ und besonders bevorzugt $\pm 2\%$ berücksichtigt wird. Bei Unterschreiten des Schwellwerts wird das Gasgemisch 3 als nicht brennbar kategorisiert und eine Signalausgabe 8 des Gaszählers 1 mit einer in Massen- oder Normvolumeneinheiten kalibrierten Skala 8b betrieben. Bei Überschreiten des Schwellwerts wird das Gasgemisch 3 als brennbar kategorisiert und eine Signalausgabe 8 des Gaszählers 1 mit einer in Energieeinheiten kalibrierten Skala 8a betrieben.

Vorzugsweise wird periodisch geprüft, ob der Gaszähler 1 mit einem brennbaren Gas 3, insbesondere Erdgas, oder mit einem nicht brennbaren Gas 3, insbesondere Stickstoff oder Luft, in Kontakt steht. Mit Vorteil werden auch Messintervalle zur Bestimmung von Sensorsignalen S; $S_m$, $S_M$, $S_E$ bei Vorhandensein eines nicht brennbaren Gasgemisches 3 gross, insbesondere 1 Minute oder länger, und bei Vorhandensein eines brennbaren Gasgemisches 3 klein, insbesondere 10 Sekunden oder kürzer, gewählt.

Ein konsumierter Gasenergiebezug kann im Gaszähler 1 aufintegriert werden und bei einer Umschaltung der Kalibration auf Massen- oder Normvolumeneinheiten zwischengespeichert und bei Rückumschaltung auf Energieeinheiten als Startwert verwendet werden. Andererseits kann die Durchflussrate $S_M$ bei einer Umschaltung der Kalibration auf Energieeinheiten weiter inkrementiert und insbesondere ausgegeben werden, oder die aufintegrierte Durchflussrate wird zwischengespeichert und insbesondere ausgegeben und bei Rückumschaltung auf Massen- oder Normvolumeneinheiten als Startwert verwendet oder als Startwert auf Null zurückgesetzt.

Mit Hilfe eines Indikators oder Displays 9 kann angezeigt werden, ob der Gaszähler 1 mit Luft oder Erdgas oder einer Mischung von Luft und Erdgas in Kontakt steht. Desweiteren können durch eine Default-Einstellung des Gaszählers 1 Massen- oder Normvolumeneinheiten angegeben werden und erst bei einem erstmaligen Kontakt mit Nutzgas, insbesondere Erdgas, Energieeinheiten angegeben werden. Auch kann durch eine Erstinitialisierung des Gaszählers 1, insbesondere bei Montage, die Kalibration automatisch von Massenoder Normvolumeneinheiten oder Luft auf Energieeinheiten oder Erdgas umgeschaltet werden. Schliesslich kann bei Kontaktnahme mit Luft, Erdgas und wiederum Luft ein Manipulationsindikator 10 des Gaszählers 1 aktiviert werden.

Die Erfindung hat auch einen Gaszähler 1 zur Ausführung des oben beschriebenen Verfahrens zum Gegenstand. Vorzugsweise haben der thermische Durchflusssensor 1a und der Gasqualitätssensor 1a einen identischen Sensoraufbau und sind insbesondere identisch. Im Gaszähler 1 werden dann die Sensorsignalwerte S; $S_m$, $S_M$, $S_E$ und ein Wärmekoeffizient $\lambda$, $c_p$, $\alpha$ des Gasgemisches 3 im selben thermischen Sensor 1a gemessen, insbesondere in einem CMOS-Anemometer 1a mit einem Heizdraht 6 und mit mindestens einem stromaufwärts angeordneten Temperatursensor 5a und optional zusätzlich mit mindestens einem stromabwärts angeordneten Temperatursensor 5b. Der thermische Durchflusssensor 1a ist als Gasqualitätssensor 1a betreibbar, wenn eine gemessene Massenflussrate einen vorgebbaren Schwellwert unterschreitet. Alternativ kann der Gasqualitätssensor 1a in einem Bereich mit konstanter Durchflussrate, insbesondere mit weitgehend ruhendem Gas 3, angeordnet sein.

Gemäss Fig. 1 umfasst der Gaszähler 1: einen Indikator oder ein Display 9 für Gasqualität, insbesondere für Vorhandenseins von Eichgas 3 oder Nutzgas 3, vorzugsweise von Luft, Erdgas oder Luft-Erdgasgemisch; einen Manipulationsindikator 10, der bei wechselnder Kontaktnahme mit einem nicht brennbaren Gas 3, insbesondere Eichgas 3, einem brennbaren Gas oder Nutzgas 3 und wiederum einem nicht brennbaren Gas 3, insbesondere einem Umgebungsgas 3, aktivierbar ist; eine Mess- und Auswerteeinheit 7 zur Bestimmung von Energieverbrauchswerten ($S_E$) und/oder Massendurchflusswerten $S_M$; und vorzugsweise separate Datenspeicher 7b, 7c zur Speicherung von Energieverbrauchswerten $S_E$ und von Massendurchflusswerten oder Normvolumenflusswerten $S_M$. Die Recheneinheit 7a umfasst auch einen Datenspeicher für bekannte Wärmekoeffizienten X, $c_p$, $\alpha$, Dichten $\rho$ oder Viskositäten $\eta$ bekannter Gase sowie Rechenmittel zum Vergleich von gemessenen mit gespeicherten oder aus Speicherwerten interpolierten Wärmekoeffizienten $\lambda$, $c_p$, $\alpha$, Dichten $\rho$ oder Viskositäten $\eta$ sowie Rechenmittel zur Bestimmung des Gasgemisches 3 als brennbar bzw. verrechenbar oder unbrennbar bzw. nicht verrechenbar.

**BEZUGSZEICHENLISTE**

| 1 | Gaszähler |
|---|---|
| 1a | thermischer Massenflusssensor, CMOS-Sensor |
| 1b | Membran |
| 2 | Strömungskanal, Rohr |
| 3 | Gas; Erdgas, Eichgas, Kalibriergas |
| 4 | Strömungsprofil |
| 5a, 5b | erster, zweiter Temperatursensor, Thermoelemente |
| 6 | Heizelement, Heizdraht |
| 7 | Mess- und Auswerteeinheit |
| 7a | Recheneinheit |
| 7b | Datenspeicher für Energieverbrauchswerte |
| 7c | Datenspeicher für Durchflusswerte |
| 8 | Signalausgabe, Display |
| 8a | Skala in Massen-/Normvolumeneinheiten kalibriert |
| 8b | Skala in Energieeinheiten kalibriert |
| 9 | Gasqualitätsindikator, Display |
| 10 | Manipulationsindikator, Display |
| CH | Erdgas, Basis-Gasgemisch |
| $f_{N2\text{-}CH}$ | Korrekturfaktor für Sensorsignal |
| F(S) | Sensoreichkurve |
| $H_{CH}$ | Heizwert, Brennwert |
| $\lambda$ | Wärmeleitfähigkeit |
| $c, c_p$ | spezifische Wärmekapazität |
| $\rho$ | Dichte |
| $\alpha$ | Diffusivität |
| $\eta$ | Viskosität |
| S | Sensorsignal |
| $S_m$ | Massenfluss(raten)signal für Eichgas oder Kalibriergas |
| $S_M$ | Massenfluss(raten)signal für Basis-Gasgemisch |
| $S_E$ | Energiewertsignal |
| $T_1, T_2$ | Temperaturen |
| $v, v_x$ | Flussgeschwindigkeit |
| dV/dT | Volumendurchflussrate |

## Patentansprüche

1. Verfahren zum Messen eines Gasverbrauchs mittels eines Gaszählers (1), insbesondere zum Messen eines verrechenbaren Gasenergiebezugs im privaten, öffentlichen oder industriellen Bereich, wobei vom Gaszähler (1) mit Hilfe eines thermischen Durchflusssensors (1a) zu einer Durchflussrate proportionale Sensorsignale (S) bestimmt werden und die Sensorsignale (S) aufgrund einer Kalibration des Gaszählers (1) als Energiemessgerät als Energiewertsignale ($S_E$) ausgegeben werden, **dadurch gekennzeichnet, dass**

    a) vom Gaszähler (1) eine Gasart insoweit bestimmt wird, dass ein nicht brennbares Gasgemisch (3) von einem brennbaren Gasgemisch (3) unterschieden wird und
    b) der Gaszähler (1) bei Vorhandensein eines nicht brennbaren Gasgemisches (3) mit einer Kalibration in Massen- oder Normvolumeneinheiten (1/min) und bei Vorhandensein eines brennbaren Gasgemisches (3) mit einer Kalibration in Energieeinheiten (kWh) betrieben wird.

2. Verfahren nach Anspruch 1, **dadurch gekennzeichnet, dass**

    a) mit Hilfe eines, thermischen Gasqualitätssensors (1a) mindestens ein gasartabhängiger Parameter ($\lambda$, c, $\alpha$, $\eta$) des Gasgemisches (3), insbesondere ein Wärmekoeffizient ($\lambda$, c, $\alpha$) wie z. B. eine Wärmeleitfähigkeit ($\lambda$) und/oder Wärmekapazität (c), bestimmt wird und
    b) durch Vergleich mit bekannten Werten des Parameters ($\lambda$, c, $\alpha$, $\eta$) für bekannte Gase oder Gasgemische das Gasgemisch (3) als brennbar oder nicht brennbar identifiziert wird.

3. Verfahren nach Anspruch 2, **dadurch gekennzeichnet, dass**

a) der thermische Durchflusssensor (1a) und der Gasqualitätssensor (1a) einen identischen Sensoraufbau haben, wobei das Gasgemisch (3) über einen ersten Temperatursensor (5a), ein Heizelement (6) und einen zweiten Temperatursensor (5b) geführt wird und

b) aus einer Differenz von Temperatursignalen der Temperatursensoren (5a, 5b) ein Massenflusssignal ($S_M$) und aus einer Summe der Temperatursignale ($T_1+T_2$) oder aus dem Temperatursignal des ersten Temperatursensors (5a) alleine ein gasartabhängiger Wärmekoeffizient ($\lambda$, c, $\alpha$) bestimmt wird.

**4.** Verfahren nach einem der vorangehenden Ansprüche, **dadurch gekennzeichnet, dass**

a) eine gemessene Wärmeleitfähigkeit ($\lambda$) auf Übereinstimmung mit einem Wärmeleitfähigkeitswert entsprechend einem Absolutwert von 0,026 W/mK für Stickstoff, Sauerstoff oder Luft, insbesondere 0,0260 W/mK für Stickstoff, 0.0263 W/mK für Sauerstoff oder 0,0261 W/mK für Luft, oder 0,0168 W/mK für Kohlendioxid getestet wird, wobei eine vorgebbare Toleranz von $\pm10\%$, bevorzugt $\pm5\%$ und besonders bevorzugt $\pm2\%$ berücksichtigt wird,

b) bei Übereinstimmung das Gasgemisch (3) als nicht brennbar kategorisiert wird und eine Signalausgabe (8) des Gaszählers (1) mit einer in Massen- oder Normvolumeneinheiten (1/min) kalibrierten Skala (8b) betrieben wird und

c) bei Nichtübereinstimmung das Gasgemisch (3) als brennbar kategorisiert wird und eine Signalausgabe (8) des Gaszählers (1) mit einer in Energieeinheiten (kWh) kalibrierten Skala (8a) betrieben wird.

**5.** Verfahren nach einem der vorangehenden Ansprüche, **dadurch gekennzeichnet, dass**

a) eine gemessene Wärmekapazität (c) mit einem Schwellwert entsprechend einem Absolutwert von 1300 J/kgK verglichen wird, wobei eine vorgebbare Toleranz von $\pm10\%$, bevorzugt $\pm5\%$ und besonders bevorzugt $\pm2\%$ berücksichtigt wird,

b) bei Unterschreiten des Schwellwerts das Gasgemisch (3) als nicht brennbar kategorisiert wird und eine Signalausgabe (8) des Gaszählers (1) mit einer in Massen- oder Normvolumeneinheiten (1/min) kalibrierten Skala (8b) betrieben wird und

c) bei Überschreiten des Schwellwerts das Gasgemisch (3) als brennbar kategorisiert wird und eine Signalausgabe (8) des Gaszählers (1) mit einer in Energieeinheiten (kWh) kalibrierten Skala (8a) betrieben wird.

**6.** Verfahren nach einem der vorangehenden Ansprüche, **dadurch gekennzeichnet, dass**

a) periodisch geprüft wird, ob der Gaszähler (1) mit einem brennbaren Gas (3), insbesondere Erdgas, oder mit einem nicht brennbaren Gas (3), insbesondere Stickstoff oder Luft, in Kontakt steht und/oder

b) Messintervalle zur Bestimmung von Sensorsignalen (S) bei Vorhandensein eines nicht brennbaren Gasgemisches (3) grpss, insbesondere 1 Minute oder länger, und bei Vorhandensein eines brennbaren Gasgemisches (3) klein, insbesondere 10 Sekunden oder kürzer, gewählt werden.

**7.** Verfahren nach einem der vorangehenden Ansprüche, **dadurch gekennzeichnet, dass** ein konsumierter Gasenergiebezug im Gaszähler (1) aufintegriert wird und bei einer Umschaltung der Kalibration auf Massen- oder Normvolumeneinheiten (1/min) zwischengespeichert und bei Rückumschaltung auf Energieeinheiten (kWh) als Startwert verwendet wird.

**8.** Verfahren nach einem der vorangehenden Ansprüche, **dadurch gekennzeichnet, dass** die Durchflussrate ($S_M$) in Massen- oder Normvolumeneinheiten (1/min) im Gaszähler (1) aufintegriert wird und

a) die Durchflussrate ($S_M$) bei einer Umschaltung der Kalibration auf Energieeinheiten (kWh) weiter inkrementiert und insbesondere ausgegeben wird oder

b) die aufintegrierte Durchflussrate zwischengespeichert und insbesondere ausgegeben wird und bei Rückumschaltung auf Massen- oder Normvolumeneinheiten (1/min) als Startwert verwendet wird oder als Startwert auf Null zurückgesetzt wird.

**9.** Verfahren nach einem der vorangehenden Ansprüche, **dadurch gekennzeichnet, dass**

a) mit Hilfe eines Indikators oder Displays (9) angezeigt wird, ob der Gaszähler (1) mit Luft oder Erdgas oder einer Mischung von Luft und Erdgas in Kontakt steht und/oder

b) durch eine Default-Einstellung des Gaszählers (1) Massen- oder Normvolumeneinheiten (1/min) angegeben werden und erst bei einem erstmaligen Kontakt mit Nutzgas, insbesondere Erdgas, Energieeinheiten (kWh) angegeben werden und/oder

c) durch eine Erstinitialisierung des Gaszählers (1), insbesondere bei Montage, die Kalibration automatisch von Massen- oder Normvolumeneinheiten (1/min) oder Luft auf Energieeinheiten (kWh) oder Erdgas umgeschaltet wird und/oder

d) bei Kontaktnahme mit Luft, Erdgas und wiederum Luft ein Manipulationsindikator (10) des Gaszählers (1) aktiviert wird.

10. Verfahren nach einem der vorangehenden Ansprüche, **dadurch gekennzeichnet, dass** für die Kalibration des Gaszählers (1) als Energiemessgerät Sensorsignale (S) in Abhängigkeit der Durchflussrate eines Eichgases (3) bestimmt und in Form einer Sensoreichkurve (F(S)) im Gaszähler (1) gespeichert werden, wobei die Sensoreichkurve (F(S)) mit einem Signal-Umrechnungsfaktor ($f_{N2-CH}$) und mit einem Heizwertfaktor ($H_{CH}$) für ein Basis-Gasgemisch (CH) korrigiert wird und das erhaltene Produkt einen Gasverbrauch in der Energieeinheit (kWh) oder einer Leistungseinheit angibt.

11. Gaszähler (1) zum Messen eines Gasverbrauchs, insbesondere eines verrechenbaren Gasenergiebezugs im privaten, öffentlichen oder industriellen Bereich, wobei der Gaszähler (1) einen thermischen Durchflusssensor (1a) aufweist und als Energiemessgerät in Energieeinheiten (kWh) kalibriert ist, **dadurch gekennzeichnet, dass**

a) der Gaszähler (1) zusätzlich als Massenflussmeter in Massen- oder Normvolumeneinheiten (1/min) kalibriert ist,

b) der Gaszähler (1) einen Gasqualitätssensor (1a) aufweist, der ein Diskriminationssignal, insbesondere einen gasartabhängigen Parameter ($\lambda$, c, $\alpha$, $\eta$), zur Unterscheidung eines brennbaren Gasgemisches (3) von einem nicht brennbaren Gasgemisch (3) erzeugt, und

c) der Gaszähler (1) aufgrund des Diskriminationssignals zwischen einem Betrieb als Energiemessgerät oder als Massenflussmeter umschaltbar ist.

12. Gaszähler (1) nach Anspruch 12, **dadurch gekennzeichnet, dass**

a) der thermische Durchflusssensor (1a) und der Gasqualitätssensor (1a) einen identischen Aufbau haben und/ oder

b) der thermische Durchflusssensor (1a) und/oder der Gasqualitätssensor (1a) CMOS-Anemometer (1a) mit einem Heizdraht (6) und stromaufwärts und stromabwärts angeordneten Temperatursensoren (5a, 5b) sind.

13. Gaszähler (1) nach einem der Ansprüche 12-13, **dadurch gekennzeichnet, dass**

a) der thermische Durchflusssensor (1a) als Gasqualitätssensor (1a) betreibbar ist, wenn eine gemessene Massenflussrate einen vorgebbaren Schwellwert unterschreitet oder

b) der Gasqualitätssensor (1a) in einem Bereich mit konstanter Durchflussrate, insbesondere mit weitgehend ruhendem Gas (3), angeordnet ist.

14. Gaszähler (1) nach einem der Ansprüche 12-14, **dadurch gekennzeichnet, dass**

a) der Gaszähler (1) einen Indikator oder ein Display (9) für Gasqualität, insbesondere für Vorhandenseins von Eichgas (3) oder Nutzgas (3), vorzugsweise von Luft, Erdgas oder Luft-Erdgasgemisch, aufweist und/oder

b) der Gaszähler (1) einen Manipulationsindikator (10) aufweist, der bei wechselnder Kontaktnahme mit einem nicht brennbaren Gas (3), insbesondere Eichgas (3), einem brennbaren Gas oder Nutzgas (3) und wiederum einem nicht brennbaren Gas (3), insbesondere einem Umgebungsgas (3), aktivierbar ist und/oder

c) der Gaszähler (1) eine Mess- und Auswerteeinheit (7) zur Bestimmung von Energieverbrauchswerten ($S_E$) und/oder Massendurchflusswerten ($S_M$) aufweist und/oder

d) der Gaszähler (1) separate Datenspeicher (7b, 7c) zur Speicherung von Energieverbrauchswerten ($S_E$) und von Massendurchflusswerten oder Normvolumenflusswerten ($S_M$) aufweist.

**Claims**

1. Method for measurement of gas consumption by means of a gas meter (1), in particular for measurement of gas energy consumption which can be billed for in the private, public or industrial field, with sensor signals (S) which are proportional to a flow rate being determined by the gas meter (1) with the aid of a thermal flow sensor (1a), and with the sensor signals (S) being emitted on the basis of a calibration of the gas meter (1) as an energy measurement device as energy value signals ($S_E$), **characterized in that**

a) the gas meter (1) determines a type of gas to such an extent that an incombustible gas mixture (3) is distinguished from a combustible gas mixture (3), and
b) in the presence of an incombustible gas mixture (3), the gas meter (1) is operated with a calibration in mass or standard volume units (1/min), and in the presence of a combustible gas mixture (3), it is operated with a calibration in energy units (kWh).

2. Method according to Claim 1, **characterized in that**

a) at least one parameter ($\lambda$, c, $\alpha$, $\eta$) of the gas mixture (3), which is dependent on the gas type, in particular a heat coefficient ($\lambda$, c, $\alpha$) such as a thermal conductivity ($\lambda$) and/or thermal capacity (c), is determined with the aid of a thermal gas quality sensor (1a), and
b) the gas mixture (3) is identified as being combustible or incombustible by comparison with known values of the parameter ($\lambda$, c, $\alpha$, $\eta$) for known gases or gas mixtures.

3. Method according to Claim 2, **characterized in that**

a) the thermal flow sensor (1a) and the gas quality sensor (la) have an identical sensor design, with the gas mixture (3) being passed via a first temperature sensor (5a), a heating element (6) and a second temperature sensor (5b), and
b) a mass flow signal ($S_M$) is determined from the difference between the signals from the temperature sensors (5a, 5b), and a heat coefficient ($\lambda$, c, $\alpha$) which is dependent on the gas type is determined solely from the sum of the temperature signals ($T_1 + T_2$) or from the temperature, signal from the first temperature sensor (5a).

4. Method according to one of the preceding claims, **characterized in that**

a) a measured thermal conductivity ($\lambda$) is tested for a match with a thermal conductivity value corresponding to an absolute value of 0.026 W/mK for nitrogen, oxygen or air, in particular 0.0260 W/mK for nitrogen, 0.0263 W/mK for oxygen or 0.0261 W/mK for air, or 0.0168 W/mK for carbon dioxide, with a predetermined tolerance of $\pm 10\%$, preferably $\pm 5\%$ and particularly preferably $\pm 2\%$ being taken into account,
b) when a match occurs, the gas mixture (3) is categorized as incombustible and a signal output (8) of the gas meter (1) is operated with a scale (8b) which is calibrated in mass or standard volume units (l/min), and
c) if no match is found, the gas mixture (3) is categorized as combustible, and a signal output (8) from the gas meter (1) is operated with a scale (8a) calibrated in energy units (kWh).

5. Method according to one of the preceding claims, **characterized in that**

a) a measured thermal capacity (c) is compared with a threshold value corresponding to an absolute value of 1300 J/kgK with a predeterminable tolerance of $\pm 10\%$, preferably $\pm 5\%$ and particularly preferably $\pm 2\%$ being taken into account,
b) if the threshold value is undershot, the gas mixture (3) is categorized as incombustible and a signal output (8) of the gas meter (1) is operated with a scale (8b) which is calibrated in mass or standard volume units (1/min), and
c) if the threshold value is overshot, the gas mixture (3) is categorized as combustible, and a signal output (8) from the gas meter (1) is operated with a scale (8a) calibrated in energy units (kWh).

6. Method according to one of the preceding claims, **characterized in that**

a) a periodic check is carried out to determine whether the gas meter (1) is in contact with a combustible gas (3), in particular natural gas, or with an incombustible gas (3), in particular nitrogen or air, and/or
b) measurement intervals for determination of sensor signals (S) are selected to be long, in particular one minute or longer, in the presence of an incombustible gas mixture (3), and are selected to be short, in particular 10 seconds or shorter, in the presence of a combustible gas mixture (3).

7. Method according to one of the preceding claims, **characterized in that** the amount of gas energy consumed is integrated in the gas meter (1) and is temporally stored on switching the calibration to the mass or standard volume units (1/min) and is used as a start value on switching back to energy units (kWh).

8. Method according to one of the preceding claims, **characterized in that** the flow rate ($S_M$) in mass cr standard volume units (1/min) is integrated in the gas meter (1), and

a) the flow rate ($S_M$) on switching the calibration to energy units (kWh) is incremented further and in particular is output, or
b) the integrated flow rate is temporarily stored and, in particular, is output, and on switching back to mass or standard volume units (1/min) is used as a start value, or is reset to zero as the start value.

9. Method according to one of the preceding claims, **characterized in that**

a) an indicator or display (9) is used to indicate whether the gas meter (1) is in contact with air or natural gas, or with a mixture of air and natural gas, and/or
b) a default setting of the gas meter (1.) indicates mass or standard volume units (1/min) and energy units (kWh) are indicated only on initial contact with useful gas, in particular natural gas, and/or
c) the calibration is automatically switched from mass or standard volume units (1/min) or air to energy units (kWh) or natural gas by a first initialization of the gas meter (1), in particular during installation, and/or
d) a manipulation indicator (10) for the gas meter (1) is activated on contact with air, natural gas and air again.

10. Method according to one of the preceding claims, **characterized in that** sensor signals (S) are determined as a function of the flow rate of a calibration gas (3) for the calibration of the gas meter (1) as an energy measurement device, and are stored in the gas meter (1) in the form of a sensor calibration curve, (F(S)), with the sensor calibration curve (F(S)) being corrected with a signal conversion factor ($f_{N2-CH}$) and with a heating value factor ($H_{CH}$) for a basic gas mixture (CH), and with the resultant product indicating gas consumption in the energy units (kWh) or in power units.

11. Gas meter (1) for measurement of gas consumption, in particular of gas energy consumption which can be billed for in the private, public or industrial field, with the gas meter (1) having a thermal flow sensor (la) and being calibrated as an energy measurement device in energy units (kWh), **characterized in that**

a) the gas meter (1) is additionally calibrated as a mass flow meter in mass or standard volume units (1/min),
b) the gas meter (1) has a gas quality sensor (la) which produces a discrimination signal, in particular a parameter ($\lambda$, c, $\alpha$, $\eta$) which is dependent on the gas type, in order to distinguish a combustible gas mixture (3) from an incombustible gas mixture (3), and
c) the gas meter (1) can be switched between operation as an energy measurement device or as a mass flow meter on the basis of the discrimination signal.

12. Gas meter (1) according to Claim 12, **characterized in that**

a) the thermal flow sensor (la) and the gas quality sensor (la) have an identical design, and/or
b) the thermal flow sensor (la) and/or the gas quality sensor (1a) are/is a CMOS anemometer (la) with a heating wire (6) and with temperature sensors (5a, 5b) arranged upstream and downstream.

13. Gas meter (1) according to one of Claims 12-13, **characterized in that**

a) the thermal flow sensor (1a) can be operated as a gas quality sensor (1a) when the measured mass flow rate is less than a predeterminable threshold value, or
b) the gas quality sensor (la) is arranged in an area with a constant flow rate, in particular with a largely stationary gas (3).

14. Gas meter (1) according to one of Claims 12-14, **characterized in that**

a) the gas meter (1) has an indicator or a display (9) for gas quality, in particular for the presence of calibration gas (3) or useful gas (3), preferably of air, natural gas or an air/natural-gas mixture, and/or
b) the gas meter (1) has a manipulation indicator (10) which can be activated in the event of alternating contact being made with an incombustible gas (3), in particular calibration gas (3), a combustible gas or useful gas (3), and once again with an incombustible gas (3), in particular a surrounding gas (3), and/or

c) the gas meter (1) has a measurement and evaluation unit (7) for- determination of energy consumption values ($S_E$) and/or mass flow values ($S_M$), and/or

d) the gas meter (1) has separate data memories (7b, 7c) for storage of energy consumption values ($S_E$) and of mass flow values or standard volume flow values ($S_M$).

**Revendications**

1. Procédé pour mesurer une consommation de gaz au moyen d'un compteur à gaz (1), en particulier pour mesurer la fourniture énergétique de gaz facturable dans le domaine privé, public ou industriel, dans lequel des signaux de capteur (S) proportionnels à un débit sont déterminés par le compteur à gaz (1) à l'aide d'un capteur de débit thermique (1a) et les signaux de capteur (S) sont délivrés sous la forme de signaux de valeur énergétique ($S_E$) en raison d'une graduation du compteur à gaz (1) comme appareil de mesure d'énergie, **caractérisé en ce que**

a) un type de gaz est déterminé par le compteur à gaz (1) de manière à faire la différence entre un mélange gazeux non combustible (3) et un mélange gazeux combustible (3) et

b) l'on fait fonctionner le compteur à gaz (1), en présence d'un mélange gazeux non combustible (3), avec une graduation en unités massiques ou volumiques standard (1/min) et, en présence d'un mélange gazeux combustible (3), avec une graduation en unités énergétiques (kWh).

2. Procédé selon la revendication 1, **caractérisé en ce que**

a) l'on détermine à l'aide d'un capteur thermique de qualité de gaz (la) au moins un paramètre ($\lambda$, c, $\alpha$, $\eta$), en fonction du type de gaz, du mélange gazeux (3), en particulier un coefficient thermique ($\lambda$, c, $\alpha$), par exemple une conductibilité thermique ($\lambda$) et/ou une capacité calorifique (c), et

b) l'on identifie le mélange gazeux (3) comme combustible ou non combustible par comparaison avec des valeurs connues du paramètre ($\lambda$, c, $\alpha$, n) pour des gaz ou des mélanges gazeux connus.

3. Procédé selon la revendication 2, **caractérisé en ce que**

a) le capteur de débit thermique (1a) et le capteur de qualité de gaz (la) ont une structure de capteur identique, le mélange gazeux (3) étant acheminé via un premier capteur de température (5a), un élément chauffant (6) et un second capteur de température (5b) et

b) l'on détermine un signal de flux massique ($S_M$) à partir d'une différence de signaux de température des capteurs de température (5a, 5b) et un coefficient thermique fonction du type de gaz ($\lambda$, c, $\alpha$) à partir d'une somme des signaux de température ($T_1+T_2$) ou du signal de température du premier capteur de température (5a) seuls.

4. Procédé selon l'une quelconque des revendications précédentes, **caractérisé en ce que**

a) l'on teste une conductibilité thermique mesurée ($\lambda$) par concordance avec une valeur de conductibilité thermique correspondant à une valeur absolue de 0,026 W/mK pour l'azote, l'oxygène ou l'air, en particulier de 0,026 W/mK pour l'azote, de 0,0263 W/mK pour l'oxygène ou de 0,0261 W/mK pour l'air ou de 0,0168 W/mK pour le dioxyde de carbone en tenant compte d'une tolérance prédéfinissable de $\pm$ 10%, de préférence de $\pm$ 5% et tout particulièrement de $\pm$ 2%,

b) en cas de concordance, le mélange gazeux (3) est classé comme non combustible et une délivrance de signal (8) du compteur à gaz (1) est effectuée sur une échelle (8b) graduée en unités massiques ou volumiques standard (1/min) et

c) en cas de non-concordance, le mélange gazeux (3) est classé comme combustible et une délivrance de signal (8) du compteur à gaz (1) est effectuée sur une échelle (8a) graduée en unités énergétiques (kWh).

5. Procédé selon l'une quelconque des revendications précédentes, **caractérisé en ce que**

a) l'on compare une capacité calorifique mesurée (c) à une valeur de seuil correspondant à une valeur absolue de 1300 J/kgK en tenant compte d'une tolérance prédéfinissable de $\pm$ 10%, de préférence de $\pm$ 5% et tout particulièrement de $\pm$ 2%,

b) en dessous de la valeur de seuil, le mélange gazeux (3) est classé comme non combustible et une délivrance de signal (8) du compteur à gaz (1) est effectuée sur une échelle (8b) graduée en unités massiques ou volumiques standard (1/min) et

c) au-dessus de la valeur de seuil, le mélange gazeux (3) est classé comme combustible et une délivrance de signal (8) du compteur à gaz (1) est effectuée sur une échelle (8a) graduée en unités énergétiques (kWh).

**6.** Procédé selon l'une quelconque des revendications précédentes, **caractérisé en ce que**

a) l'on vérifie périodiquement si le compteur à gaz (1) est mis en contact avec un gaz combustible (3), en particulier du gaz naturel, ou avec un gaz non combustible (3), en particulier de l'azote ou de l'air, et/ou,

b) pour déterminer les signaux de capteur (S), on choisit de grands intervalles de mesure en présence d'un mélange gazeux non combustible (3), en particulier 1 minute ou plus, et de petits intervalles de mesure en présence d'un mélange gazeux combustible (3), en particulier 10 secondes ou moins.

**7.** Procédé selon l'une quelconque des revendications précédentes, **caractérisé en ce qu'**une fourniture énergétique de gaz consommée est intégrée au compteur à gaz (1) et y est stockée provisoirement lors d'un passage de la graduation à des unités massiques ou volumiques standard (1/mn) et est utilisée comme valeur de départ lors d'un retour à des unités énergétiques (kWh).

**8.** Procédé selon l'une quelconque des revendications précédentes, **caractérisé en ce que** le débit ($S_M$) est intégré au compteur à gaz (1) en unités massiques ou volumiques standard (1/min) et

a) le débit ($S_M$) est ensuite incrémenté et en particulier délivré lors d'un passage de la graduation à des unités énergétiques (kWh) et

b) le débit intégré est stocké provisoirement et en particulier délivré et est utilisé comme valeur de départ lors d'un retour à des unités massiques ou volumiques standard (1/min) ou est remis à zéro comme valeur de départ.

**9.** Procédé selon l'une quelconque des revendications précédentes, **caractérisé en ce que**

a) à l'aide d'un indicateur ou d'un affichage (9), on indique si le compteur à gaz (1) est en contact avec de l'air ou avec du gaz naturel ou avec un mélange d'air et de gaz naturel et/ou

b) des unités massiques ou volumiques standard (1/min) sont indiquées par un réglage par défaut du compteur à gaz (1) et ce n'est que lors d'un premier contact avec un gaz utile, en particulier du gaz naturel, que sont indiquées des unités énergétiques (kWh), et/ou

c) par une première initialisation du compteur à gaz (1), en particulier lors du montage, la graduation passe automatiquement d'unités massiques ou volumiques standard (1/min) ou de l'air à des unités énergétiques (kWh) ou du gaz naturel, et/ou

d) lors d'un contact avec de l'air, du gaz naturel et à nouveau de l'air, un indicateur de manipulation (10) du compteur à gaz (1) est activé.

**10.** Procédé selon l'une quelconque des revendications précédentes, **caractérisé en ce que**, pour la graduation du compteur à gaz (1) comme appareil de mesure d'énergie, on détermine des signaux de capteur (S) en fonction du débit d'un gaz d'étalonnage (3) et on les stocke sous la forme d'une courbe d'étalonnage de capteur (F(S)) dans le compteur à gaz (1), la courbe d'étalonnage de capteur (F(S)) étant corrigée par un facteur de conversion de signal ($f_{N2-CH}$) et par un facteur de pouvoir calorifique ($H_{CH}$) pour un mélange gazeux de base (CH) et le produit obtenu indiquant une consommation de gaz en unité énergétique (kWh) ou en unité de puissance.

**11.** Compteur à gaz (1) pour mesurer une consommation de gaz, en particulier une fourniture énergétique de gaz facturable dans le domaine privé, public ou industriel, dans lequel le compteur à gaz (1) présente un capteur de débit thermique (1a) et est gradué comme appareil de mesure d'énergie en unités énergétiques (kWh), **caractérisé en ce que**

a) le compteur à gaz (1) est en plus gradué comme fluxmètre massique en unités massiques ou volumiques standard (1/min),

b) le compteur à gaz (1) présente un capteur de qualité de gaz (1a) qui produit un signal de discrimination, en particulier un paramètre ($\lambda$, c, $\alpha$, $\eta$) fonction du type de gaz pour distinguer un mélange gazeux combustible (3) d'un mélange gazeux non combustible (3), et

c) le compteur à gaz (1) peut, en raison du signal de discrimination, permuter entre un fonctionnement comme appareil de mesure d'énergie et comme fluxmètre massique.

**12.** Compteur à gaz (1) selon la revendication 11, **caractérisé en ce que**

a) le capteur de débit thermique (1a) et le capteur de qualité de gaz (1a) ont une structure identique et/ou
b) le capteur de débit thermique (1a) et/ou le capteur de qualité de gaz (la) sont des anémomètres CMOS (la) avec un fil chauffant (6) et des capteurs de température (5a, 5b) agencés en amont et en aval du flux.

**13.** Compteur à gaz (1) selon l'une quelconque des revendications 11 à 12, **caractérisé en ce que**

a) le capteur thermique de débit (la) peut fonctionner comme capteur de qualité de gaz (la) lorsqu'un débit massique mesuré est en dessous d'une valeur de seuil prédéfinissable ou
b) le capteur de qualité de gaz (la) est agencé dans une zone à débit constant, en particulier avec du gaz largement statique (3).

**14.** Compteur à gaz (1) selon l'une quelconque des revendications 11 à 13, **caractérisé en ce que**

a) le compteur à gaz (1) présente un indicateur ou un affichage (9) pour la qualité du gaz, en particulier pour la présence de gaz d'étalonnage (3) ou de gaz utile (3), de préférence d'air, de gaz naturel ou d'un mélange d'air et de gaz naturel, et/ou
b) le compteur à gaz (1) présente un indicateur de manipulation (10), qui peut être activé lors d'une mise en contact alternée avec un gaz non combustible (3), en particulier du gaz d'étalonnage (3), un gaz combustible ou du gaz utile (3) et à nouveau un gaz non combustible (3), en particulier un gaz ambiant (3), et/ou
c) le compteur à gaz (1) présente une unité de mesure et d'exploitation (7) pour déterminer des valeurs de consommation d'énergie ($S_E$) et/ou des valeurs de débit massique ($S_M$), et/ou
d) le compteur à gaz (1) présente des mémoires de données (7b, 7c) pour stocker des valeurs de consommation d'énergie ($S_E$) et des valeurs de débit massiques ou volumiques standard ($S_M$).

**FIG. 1**

dV/dt [ml/min]

**FIG. 2**

Fig. 3

| Temperature: 300K; Pressure: 1.013 bar | | | | |
|---|---|---|---|---|
| Gas | $\lambda$ | $c_p$ | $\rho$ | $\alpha$ |
| | W/(mK) | J/(kgK) | kg/m$^3$ | m$^2$/s |
| Methane | 0.0341 | 2200.34 | 0.653 | 2.37E-05 |
| Ethane | 0.0213 | 1749.25 | 1.231 | 9.90E-06 |
| Propane | 0.0180 | 1626.57 | 1.967 | 5.63E-06 |
| Carbon dioxide | 0.0168 | 842.99 | 1.797 | 1.11E-05 |
| Nitrogen | 0.0260 | 1038.77 | 1.138 | 2.20E-05 |
| Oxygen | 0.0263 | 918.78 | 1.301 | 2.20E-05 |
| Hydrogen | 0.1869 | 14285.71 | 0.082 | 1.60E-04 |
| Water | 0.0187 | 1865.11 | 0.767 | 1.31E-05 |
| Carbon monoxide | 0.0250 | 1038.91 | 1.138 | 2.11E-05 |
| Helium | 0.1567 | 5196.10 | 0.163 | 1.86E-04 |

Fig. 4